# EUROPEAN PATENT SPECIFICATION

(11) **EP 2 609 429 B1**
(45) Date of publication and mention of the grant of the patent: **20.09.2017**
(21) Application number: 11820697.8
(22) Date of filing: 26.08.2011
(51) Int. Cl.: G01N 33/53, G01N 33/48

(54) **METHODS FOR DETECTING ANTI-HE4 ANTIBODIES AND METHODS OF DIAGNOSIS AND/OR PROGNOSIS OF CONDITIONS ASSOCIATED WITH HE4-EXPRESSING CELLS**
VERFAHREN ZUM NACHWEIS VON ANTI-HE4-ANTIKÖRPERN UND VERFAHREN ZUR DIAGNOSE UND/ODER PROGNOSE VON MIT HE4-EXPRIMIERENDEN ZELLEN ASSOZIIERTEN LEIDEN
MÉTHODES POUR LA DÉTECTION D'ANTICORPS ANTI-HE4 ET MÉTHODES DE DIAGNOSTIC ET/OU DE PRONOSTIC D'ÉTATS ASSOCIÉS À DES CELLULES EXPRIMANT HE4

(30) Priority: 26.08.2010 US 377387 P
(43) Date of publication of application: 03.07.2013
(73) Proprietor: University of Washington through its Center for Commercialization, Seattle, WA 98105-4608 (US)
(72) Inventor: HELLSTROM, Karl, Erik, Seattle, WA 98105 (US); HELLSTROM, Ingegerd, Seattle, WA 98105 (US); LIU, Pu, Rockville, MD (US); JAFFAR, Jade, Camperdown, New South Wales (AU); SWISHER, Elizabeth, Seattle, WA 98105 (US)
(74) Representative: HGF Limited
(86) International application number: PCT/US2011/049274
(87) International publication number: WO 2012/027631

(56) References cited:
- WO-A2-03/021273
- WO-A2-2007/081767
- US-A1- 2002 182 619
- US-A1- 2003 108 965
- INGEGERD HELLSTROM ET AL: "Anti-HE4 antibodies in infertile women and women with ovarian cancer", GYNECOLOGIC ONCOLOGY, vol. 130, no. 3, 1 September 2013 (2013-09-01), pages 629-633, XP055100658, ISSN: 0090-8258, DOI: 10.1016/j.ygyno.2013.05.028
- INGEGERD HELLSTROM ET AL: "Two new biomarkers, mesothelin and HE4, for diagnosis of ovarian carcinoma", EXPERT OPINION ON MEDICAL DIAGNOSTICS, vol. 5, no. 3, 1 May 2011 (2011-05-01), pages 227-240, XP055052244, ISSN: 1753-0059, DOI: 10.1517/17530059.2011.559459
- HELLSTROM I ET AL: "The HE4 (WFDC2) protein is a biomarker for ovarian carcinoma", CANCER RESEARCH, AMERICAN ASSOCIATION FOR CANCER RESEARCH, US, vol. 63, no. 13, 1 July 2003 (2003-07-01), pages 3695-3700, XP002290876, ISSN: 0008-5472
- JI QIU ET AL: "Autoantibody Profiling for Cancer Detection", CLINICS IN LABORATORY MEDICINE, vol. 29, no. 1, 1 March 2009 (2009-03-01), pages 31-46, XP055100749, ISSN: 0272-2712, DOI: 10.1016/j.cll.2009.01.002
- HELLSTROM INGEGERD ET AL: "SMRP and HE4 as biomarkers for ovarian carcinoma when used alone and in combination with CA125 and/or each other", ADVANCES IN EXPERIMENTAL MEDICINE AND BIOLOGY, SPRINGER, US, 1 January 2008 (2008-01-01), pages 15-21, XP009111346, ISSN: 0065-2598, DOI: 10.1007/978-0-387-68969-2_2
- HAVRILESKY ET AL.: 'Evaluation of biomarker panels for early stage ovarian cancer detection and monitoring for disease recurrence' GYNECOL ONCOL. vol. 110, no. 3, September 2008, pages 374 - 382, XP024529692

## Description

### CROSS-REFERENCE TO RELATED APPLICATIONS

This application claims the benefit of the filing date of U.S. Provisional Application No. 61/377,387, which was filed on August 26, 2010.

### FIELD OF THE INVENTION

The present invention relates to methods for assessing the risk, diagnosis and/or prognosis of subjects at risk from or suffering from a cancer associated with human epididymal four-disulfide core protein ("HE4") expressing tumors and, in particular, to methods of measuring anti-HE4 antibodies for use as an indicator of the presence of HE4 expressing cells, such as ovarian tumor cells, and/or to determine the clinical status of a patient undergoing treatment for a cancer associated with one or more HE4 expressing tumors.

### BACKGROUND

Ovarian carcinoma (OvC) is the second most frequent and the most lethal gynecologic malignancy in the western world. Most cases are diagnosed at an advanced stage, and this is reflected by a poor prognosis with the overall five-year survival rate not exceeding 35%. Ovarian carcinoma is disproportionately deadly because symptoms are vague and non-specific. Ovarian cancers shed malignant cells into the naturally occurring fluid within the abdominal cavity. These cells then have the potential to float in this fluid and frequently implant on other abdominal (peritoneal) structures including the uterus, urinary bladder, bowel, and lining of the bowel wall (omentum). These cells can begin forming new tumor growths before cancer is even suspected. More than 60% of patients presenting with this disease already have stage III or stage IV disease, when it has already spread beyond the ovaries, and more than 75% of these patients die from disease, in spite of recent improvements of chemotherapy for ovarian cancer. However, if diagnosis is made early in the disease, five-year survival rates can reach 90% to 98%.

One marker for ovarian cancer that is used in serum assays for ovarian cancer is CA125 (Bast, R.C., et al., Gynecol. Oncol. 22:115-120 (1985); Einhorn, N., et al., Obstet. Gynaecol. 67:414-416 (1986); Einhorn, et al., Obstet. Gynecol. 80:14-18 (1992); Jacobs, I.J., et al., Br. Med. J. 313:1355-1358 (1996)). However, while CA125 is found elevated in the majority of all ovarian cancers, it is found in only half of those with early stage disease (Hellstrom, I., et al., Cancer Research 63:3695-3700 (2003)). Moreover, CA125 is also elevated in several non-malignant conditions (Fung, M.F., et al., J. Obstet. Gynaecol. Can., 26:717-728 (2004); Mas, M.R., et al, Dig. Liver Dis. 32:595-597 (2000); Malkasian, G.D., et al, Am. J. Obstet. Gynecol. 159:341-346 (1988)), which can lead to a false positive result.

Thus, there is a great need to develop more effective tools for detecting potentially curable, early stage malignant conditions, such as ovarian carcinoma.

### SUMMARY

In accordance with the foregoing, in one aspect, a method is provided for detecting the presence of HE4-expressing cells in a human subject comprising determining the presence or amount of anti-HE4 antibodies in a biological sample obtained from the human subject, wherein the presence or amount of anti-HE4 antibodies in the biological sample is indicative of the presence of HE4-expressing cells in the human subject. In some embodiments, the presence of HE4-expressing cells in a human subject is indicative that the subject is suffering from ovarian cancer, or is at risk for developing a ovarian tumor.

In another aspect, a method is provided for monitoring the efficacy of treatment of a human cancer patient undergoing therapeutic treatment for an HE4-expressing tumor. The method comprises: (a) providing a biological sample from a human patient undergoing therapeutic treatment for a cancer associated with HE4-expressing tumor cells; (b) determining the presence or amount of anti-HE4 antibodies in the biological sample by contacting the biological sample with a polypeptide encoded by a polynucleotide that selectively hybridizes to a sequence at least 90% identical to a sequence comprising at least 20 contiguous nucleotides of SEQ ID NO:1; and (c) comparing the determined presence or amount of anti-HE4 antibodies to an antibody reference standard, wherein an amount of anti-HE4 antibody greater than the reference standard is indicative of a positive response to the therapeutic treatment for the cancer.

In another aspect, a kit is provided for detecting the presence of HE4-expressing cells in a human subject, the kit comprising reagents specific for detection of the presence or amount of anti-HE4 antibodies in a biological sample obtained from a human subject and printed instructions for comparison of the detected presence or amount of anti-HE4 antibodies with a reference standard.

### DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:
FIGURE 1 graphically illustrates the results of an ELISA assay testing for the presence/amount of anti-HE4 antibodies from human serum samples obtained from 10 patients with ovarian cancer, and 1 healthy control subject; as described in Example 2.
FIGURE 2 depicts HE4 fragments expressed as fusion proteins with coat protein pVIII using the primers shown in Table1.

### DETAILED DESCRIPTION

Unless specifically defined herein, all terms used herein have the same meaning as they would to one skilled in the art of the present invention.

The terms "percent identity" or "percent identical," as applied to polypeptide sequences, such as the HE4 polypeptide or a portion thereof, is defined as the percentage of amino acid residues in a candidate protein sequence that are identical with the subject protein sequence (such as the amino acid sequence set forth in SEQ ID NO:2, or a portion thereof comprising at least 10 consecutive amino acid residues) after aligning the candidate and subject sequences to achieve the maximum percent identity. For example, percentage identity between two protein sequences can be determined by pairwise comparison of the two sequences using the bl2seq interface at the Web site of the National Center for Biotechnology Information (NCBI), U.S. National Library of Medicine, 8600 Rockville Pike, Bethesda, Maryland 20894, U.S.A. The bl2seq interface permits sequence alignment using the BLAST tool described by Tatiana, A., et al, "Blast 2 Sequences--A New Tool for Comparing Protein and Nucleotide Sequences," FEMS Microbiol. Lett. 174:247-250 (1999). The following alignment parameters are used: Matrix = BLOSUM62; Gap open penalty = 11; Gap extension penalty = 1; Gap x_dropff = 50; Expect = 10.0; Word size = 3; and Filter = off.

The terms "percent identity" or "percent identical," as applied to nucleic acid molecules, is the percentage of nucleotides in a candidate nucleic acid sequence that are identical with a subject nucleic acid molecule sequence (such as the nucleic acid molecule sequence set forth in SEQ ID NO: 1, or a portion thereof comprising at least 20 consecutive nucleotides) after aligning the sequences to achieve the maximum percent identity, and not considering any nucleic acid residue substitutions as part of the sequence identity. No gaps are introduced into the candidate nucleic acid sequence in order to achieve the best alignment. Nucleic acid sequence identity can be determined in the following manner. The subject polynucleotide molecule sequence is used to search a nucleic acid sequence database, such as the Genbank database, using the program BLASTN version 2.1 (based on Altschul, et al., Nucleic Acids Research 25:3389-3402 (1997)). The program is used in the ungapped mode. Default filtering is used to remove sequence homologies due to regions of low complexity as defined in Wootton, J.C., and S. Federhen, Methods in Enzymology 266:554-571 (1996). The default parameters of BLASTN are utilized.

As used herein, the term "healthy human subject" refers to an individual who is known not to suffer from cancer, such knowledge being derived from clinical data on the individual including, but not limited to, a different cancer assay to that described herein. The healthy individual is also preferably asymptomatic with respect to the early symptoms associated with HE4-expressing tumors such as ovarian cancer, which include, for example, rectal pressure, abdominal bloating, and swelling; and is also preferably asymptomatic with respect to other reproductive diseases or conditions.

As used herein, the term "HE4-expressing tumor" refers to any type of cancer cells and/or tumors that are identified as having a neoplastic condition associated with an increased expression of HE4, wherein HE4 refers to at least one of SEQ ID NO:1, SEQ ID NO:2 and mammalian homologs thereof, or a fragment thereof comprising at least ten consecutive residues of the protein (SEQ ID NO:2), or at least 20 consecutive nucleotides of the cDNA (SEQ ID NO:1), as compared to normal tissues, including but not limited to, ovarian cancer, lung adenocarcinoma (Bingle et al., Respiratory Research 7:61-80 (2006), and salivary gland tumors.

As used herein, the term "ovarian cancer" refers to any type of ovarian cancer including, but not limited to, serous ovarian cancer, non-invasive ovarian cancer, mixed phenotype ovarian cancer, mucinous ovarian cancer, endometrioid ovarian cancer, clear cell ovarian cancer, papillary serous ovarian cancer, Brenner cell, and undifferentiated adenocarcinoma.

As used herein, the term "recurrence of a tumor expressing HE4" refers to clinical evidence of cancer related to cells expressing HE4, for example, ovarian cancer, or tumor cells derived therefrom based upon clinical data on the individual including, but not limited to, a different cancer assay to that described herein.

As used herein, the term "good prognosis" in the context of cancer associated with one or more HE4-expressing tumors (e.g., ovarian cancer) refers to patients who are likely to be cured from their disease, or to have at least a five-year tumor-free survival period following the initial diagnosis.

As used herein, the term "poor prognosis" in the context of cancer associated with one or more HE4-expressing tumors (e.g., ovarian cancer) refers to patients who are likely to die from their disease within a five-year period following the initial diagnosis.

The human epididymal four-disulfide core protein ("HE4") (SEQ ID NO:2) is encoded by the mRNA sequence set forth as SEQ ID NO:1 (which corresponds to Genbank Accession No. AY212888). HE4 cDNA was first isolated from human epididymis (Kirchhoff et al., 1991), and HE4 cDNA was later detected with high frequency in cDNA libraries constructed from ovarian carcinomas (Wang et al., Gene 229:101 (1999)). The HE4 protein belongs to the "four-disulfide core" family of proteins, which comprises a heterogeneous group of small acid and heat stable molecules of divergent function, referred to as "soluble HE4-related peptides" (SHRP) (Kirchhoff, et al., Biol. Reprod. 45:350-357 (1991). The conserved spacing of eight core cysteine residues in the amino acid sequences of the four-disulfide core family member polypeptides (aa regions 32-73 and 76-123 of SEQ ID NO:2) is thought to direct the folding of these molecules into a compact and stable structure. Many members of the four-disulfide core family are protease inhibitors; however, for some family members, including HE4, no function has yet been identified.

As used herein, the term "HE4" protein encompasses naturally occurring HE4 protein that is isolated from a biological sample obtained from a human subject as well as HE4 protein isolated from cultured cells making HE4 (e.g., cultured ovarian carcinoma cells), or made by recombinant DNA technology (e.g., in eukaryotic expression systems (e.g., COS cells)), in yeast, mammalian, or in bacterial expression systems.

In accordance with the foregoing, the present inventors have generated a reproducible assay for detecting antibodies to native HE4 protein (SEQ ID NO:2) in a biological sample (e.g., plasma or serum), as described in Example 2. As further described in Examples 2-3, the inventors have used this assay to detect the presence of antibodies to HE4 in human subjects, and have found that subjects subjects clinically identified as suffering from ovarian cancer had a significantly higher incidence of anti-HE4 antibodies as compared to normal healthy female subjects.

In accordance with the foregoing, in one aspect, a method is provided for detecting the presence of HE4-expressing cells in a human subject. The method comprises determining the presence or amount of anti-HE4 antibodies in a biological sample obtained from the human subject, wherein the presence or amount of anti-HE4 antibodies in the biological sample is indicative of the presence of HE4-expressing cells in the human subject. In one embodiment, the presence or amount of anti-HE4 antibodies in the biological sample is determined by contacting the biological sample with a polypeptide encoded by a polynucleotide that selectively hybridizes to a sequence at least 80% identical, or at least 90% identical, or at least 95% identical, or at least 98% identical, or at least 99% identical to a sequence comprising at least 20 contiguous nucleotides of SEQ ID NO:1. In some embodiments, the presence or amount of anti-HE4 antibody in the biological sample is determined by contacting the biological sample with a polypeptide comprising a sequence at least 80% identical, or at least 90% identical, or at least 95% identical, or at least 98% identical, or at least 99% identical to a sequence comprising at least 10 contiguous amino acids of SEQ ID NO:2. In one embodiment, the presence or amount of anti-HE4 antibodies in comparison to a reference standard (e.g., a negative control) is indicative of the presence of HE4-expressing cells, such as tumor cells, in the human subject. In another embodiment, the amount of anti-HE4 antibodies over a predetermined threshold amount is indicative of the presence of HE4-expressing cells in a human subject. In some embodiments of the method, the presence of HE4-expressing cells in a human subject is indicative that the subject is suffering from or at risk for developing ovarian cancer.

A wide variety of biological samples may be used in the methods of the invention, including biological fluids. Non-limiting examples of biological fluids include blood, plasma, serum, ascitic fluid, urine, saliva, tears, pleural fluid, sputum, vaginal fluid (discharge), and washings obtained during a medical procedure (e.g., pelvic or other washings obtained during biopsy, endoscopy, or surgery).

The methods of this aspect of the invention may be used as a diagnostic tool to distinguish between a subject suffering from a disease or condition associated with the expression of HE4 and a disease or condition not associated with the expression of HE4. In one embodiment of the method, a biological sample is obtained from a human subject suffering from at least one symptom associated with ovarian cancer. Symptoms associated with ovarian cancer are known to those of skill in the field of medicine. Non-limiting examples of such symptoms include abdominal swelling/bloating, abdominal/pelvic pain or pressure, gastrointestinal symptoms (e.g., gas, indigestion, nausea, or changes in bowel movements), vaginal bleeding or discharge, urinary problems (e.g., urgency, burning or spasms), fatigue, fever, back pain, and difficulty breathing.

In some embodiments, the methods of this aspect further comprise performing at least one additional diagnostic assay to determine if the subject with anti-HE4 antibodies has an ovarian tumor. In some embodiments, the methods of this aspect of the invention further comprise performing at least one additional diagnostic assay for ovarian cancer on the subject, such as, for example, detecting the presence of CA125 in a biological sample, ultrasound, CT scan, MRI scan, biopsy, aspirate, and the like.

As described in more detail herein, in some embodiments, the methods that include the detection of antibodies to native HE4 may be used and optionally combined with an assay to detect SHRP antigen, in order to detect the presence of HE4-expressing tumor cells, to determine the presence or likelihood of recurrence of a cancer associated with an HE4-expressing tumor, such as ovarian cancer, to assess the clinical status and/or prognosis of a patient suffering from a cancer associated with HE4-expressing tumors, and/or to monitor the efficacy of treatment of cancer in a patient. The amount of SHRP antigen detected in the biological sample may be compared to a reference standard such as an antigen reference value, wherein detection of an increased amount of SHRP antigen in the sample as compared to the reference standard is indicative of the presence of HE4-expressing tumor cells in the human subject.

In another embodiment, a method is provided for monitoring the efficacy of treatment of a human cancer patient undergoing therapeutic treatment for an HE4-expressing tumor. The method comprises: (a) providing a biological sample from a human patient undergoing therapeutic treatment for a cancer associated with an HE4-expressing tumor; (b) determining the presence or amount of anti-HE4 antibodies in the biological sample by contacting the biological sample with a polypeptide encoded by a polynucleotide that selectively hybridizes to a sequence at least 90% identical to a sequence comprising at least 20 contiguous nucleotides of SEQ ID NO:1; and (c) comparing the determined presence or amount of anti-HE4 antibodies to an antibody reference value wherein an amount of anti-HE4 antibody greater than the antibody reference value is indicative of a positive response to the therapeutic treatment for the cancer.

In another embodiment, a method is provided for determining the likelihood of recurrence of an HE4-expressing tumor in a human patient undergoing therapeutic treatment for a cancer associated with an HE4-expressing tumor. The method comprises: (a) providing a biological sample from a human patient undergoing therapeutic treatment for a cancer associated with an HE4-expressing tumor; (b) determining the presence or amount of anti-HE4 antibodies in the biological sample by contacting the biological sample with a polypeptide encoded by a polynucleotide that selectively hybridizes to a sequence at least 80%, such as at least 90% identical to a sequence comprising at least 20 contiguous nucleotides of SEQ ID NO:1; and (c) comparing the presence or amount of anti-HE4 antibodies determined in step (b) to an antibody reference value, wherein an amount of anti-HE4 antibody greater than the antibody reference value is indicative of a lower risk of HE4-expressing tumor recurrence and wherein an amount of anti-HE4 antibody lower than the reference value is indicative of greater risk of HE4-expressing tumor recurrence in the human patient.

In accordance with one embodiment of the methods, a human patient undergoing therapeutic treatment for a cancer associated with an HE4-expressing tumor is assessed for their clinical status and likelihood of recurrence of cancer. The methods in accordance with this embodiment may be practiced with patients previously diagnosed and treated for an HE4-expressing tumor, such as ovarian cancer, lung adenocarcinoma, or a salivary gland tumor (e.g., treated with surgery and/or previously or currently undergoing therapeutic treatment, such as chemotherapy, radiation therapy, protein therapeutics, including antibodies, gene therapy, cancer vaccine therapy, stem cell transplant, or other therapy). Recurrence of ovarian cancer is a clinical recurrence as determined by the presence of one or more clinical symptoms of an ovarian cancer, such as, for example, a metastases, or alternatively, as determined in a biochemical test, immunological test, or serological test such as, for example, a cross-reactivity in a biological sample to a CA125 antibody, or other diagnostic test. Preferably, the recurrence of ovarian cancer is capable of being detected at least about 2 years from treatment, more preferably about 2-3 years from treatment, and even more preferably, about 4 or 5 or 10 years from treatment.

A 1-4 staging system is used for describing ovarian cancer, as set forth by the International Federation of Gynecology and Obstetrics ("FIGO") staging system, which uses information obtained after surgery. Surgeries can include a total abdominal hysterectomy, removal of one or both ovaries and fallopian tubes, the omentum, and/or pelvic washings for cytology.

### Stage I - limited to one or both ovaries

IA - involves one ovary; capsule intact; no tumor on ovarian surface; no malignant cells in ascites or peritoneal washings
IB - involves both ovaries; capsule intact; no tumor on ovarian surface; negative washings
IC - tumor limited to ovaries with any of the following: capsule ruptured, tumor on ovarian surface, positive washings

### Stage II - pelvic extension or implants

IIA - extension or implants onto uterus or fallopian tube; negative washings
IIB - extension or implants onto other pelvic structures; negative washings
IIC - pelvic extension or implants with positive peritoneal washings

### Stage III - microscopic peritoneal implants outside of the pelvis; or limited to the pelvis with extension to the small bowel or omentum

IIIA - microscopic peritoneal metastases beyond pelvis
IIIB - macroscopic peritoneal metastases beyond pelvis less than 2 cm in size
IIIC - peritoneal metastases beyond pelvis > 2 cm or lymph node metastases, note: para-aortic lymph node metastases are considered regional lymph nodes

### Stage IV - distant metastases--in the liver, or outside the peritoneal cavity

In accordance with some embodiments, a biological sample is obtained from a human patient (previously diagnosed with and previously treated for ovarian cancer, or currently undergoing treatment for ovarian cancer) and is assayed for the presence or concentration of anti-HE4 antibodies. Biological samples for use in the methods of the invention include biological fluids. Non-limiting examples of biological fluids include blood, plasma, serum, ascitic fluid, urine, saliva, tears, pleural fluid, sputum, vaginal fluid (discharge), and washings obtained during a medical procedure (e.g., pelvic or other washings obtained during biopsy, endoscopy, or surgery). The ability to use a sample of biological fluid to assess the clinical status of a subject with regard to an HE4-expressing tumor (such as ovarian cancer or other HE4-expressing tumors), provides relative ease as compared to obtaining a tissue biopsy sample of a tumor. Moreover, it enables monitoring of a patient during and/or post-treatment and, importantly, allows for earlier detection of recurrence and/or progression of ovarian cancer (or other HE4-expressing tumors).

In accordance with the methods of this aspect of the invention, the concentration of anti-HE4 antibody is measured in a biological sample obtained from a human patient. Any immunoassay may be used to measure the concentration of anti-HE4 antibody; for example, enzyme-linked immunosorbent assays (ELISA) and radioimmunoassays (RIA), western blotting, FACS analysis, and the like. More preferably, the assay will be capable of generating quantitative results. The biological sample may be diluted in a suitable buffer prior to analysis, for example, the sample may be diluted by a factor of at least 1:2, 1:5, 1:10, 1:20, 1:30, 1:40, 1:50, 1:80, 1:100, 1:200 or greater.

In one embodiment, the presence or amount of anti-HE4 antibody in the biological sample is determined by contacting the biological sample with an HE4 polypeptide encoded by a polynucleotide that selectively hybridizes to a sequence at least 80% identical (e.g., at least 85% identical, or at least 90% identical, or at least 95% identical, or at least 99% identical) to SEQ ID NO:1, or a fragment thereof comprising at least 20 consecutive nucleotides (or at least 25 or 30, or at least 40, 60, or 80 consecutive nucleotides) of SEQ ID NO:1.

In another embodiment, the presence or amount of anti-HE4 antibody in the biological sample is determined by contacting the biological sample with an HE4 polypeptide at least 80% identical (e.g., at least 85% identical, or at least 90% identical, or at least 95% identical, or at least 99% identical) to the human soluble HE4-related protein provided as SEQ ID NO:2, or a fragment thereof comprising at least 10 consecutive amino acid residues, (or at least 20 or at least 30, such as at least 50 consecutive amino acid residues) of SEQ ID NO:2.

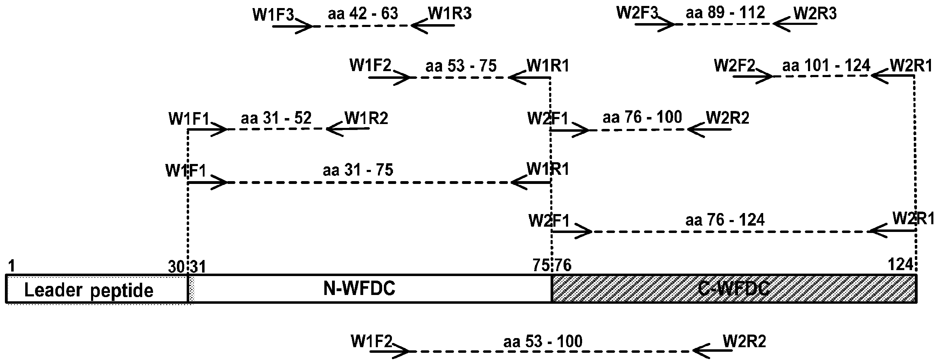

A fragment of an HE4 polypeptide has a amino acid sequence contains fewer amino acids than the full-length HE4 amino acid sequence as set forth in SEQ ID NO: 2. In some embodiments, the fragment can include the N-terminal domain, N-WFDC. As shown in Figure 2, N-WFDC (SEQ ID NO: 5) extends from amino acid 31 to amino acid 75 of SEQ ID NO: 2. In some embodiments, the fragment can include the C-terminal domain C-WFDC. As shown in Figure 2, C-WFDC (SEQ ID NO: 6) extends from amino acid 76 to amino acid 124 of SEQ ID NO: 2. Other exemplary fragments of HE4 can include polypeptides having an amino acid sequence corresponding to the amino acid sequence extending from positions 31-52 (SEQ ID NO: 7); positions 42-63 (SEQ ID NO: 8); positions 53-75 (SEQ ID NO: 9); positions 76-100 (SEQ ID NO: 10); positions 89-112 (SEQ ID NO: 11); positions 89-102 (SEQ ID NO: 12); or positions 101-124 (SEQ ID NO: 13) of SEQ ID NO: 2. In some embodiments, fragments of HE4 can span the N-WFDC and the C-WFDC domains. Exemplary fragments include polypeptides having an amino acid sequence corresponding to the amino acid sequence extending from positions 53-100 (SEQ ID NO: 14) of SEQ ID NO: 2. A fragment comprising at least 20 consecutive nucleotides (or at least 25 or 30, or at least 40, 60, or 80 consecutive nucleotides) of SEQ ID NO:1 can be, for example, a fragment of SEQ ID NO: 1 that encodes SEQ ID NO: 5-13.

The anti-HE4 antibodies specifically bind to an epitope on the HE4 polypeptide. An epitope refers to an antigenic determinant on a target that is specifically bound by the paratope, i.e., the binding site of an antibody. Epitopic determinants usually consist of chemically active surface groupings of molecules such as amino acids or sugar side chains, and typically have specific three-dimensional structural characteristics, as well as specific charge characteristics. Epitopes generally have between about 4 to about 10 contiguous amino acids (a continuous epitope), or alternatively can be a set of noncontiguous amino acids that define a particular structure (e.g., a conformational epitope). Thus, an epitope can consist of at least 4, at least 6, at least 8, at least 10, and at least 12 such amino acids. Methods of determining the
coding regions indicated in Figure x. In the 5 '-ends were restriction sites for Hindlll and Pstl inserted for cloning in fusion with the pVIII signal peptide and the pVIII mature coat protein.

Table 3. PCR primers (SEQ ID NOS 15-26, respectively, in order of appearance) used for amplification of HE4 fragments
Primer Sequence 5' to 3 WAP
W1F1 TGCTAAGCTTTGCC GAGAAGACTGGCGTGTGCCC N-WAP
W1F2 TGCTAAGCTTTGCC AGCGAATGCGCCGACAACC N-WAP
W1F3 TGCTAAGCTTTGCC GACCAGAACTGCACGCAAG N-WAP
W1R1 CCTTCTGCAGG ATCATTGGGCAGAGAGCAG N-WAP
W1R2 CCTTCTGCAGG GTCCGAGACGCACTCTTGC N-WAP
W1R3 CCTTCTGCAGG GCTGCAGCACTTGAGGTTG N-WAP
W2 F 1 TGCTAAGCTTTGCC AAGGAGGGTTCCTGCCCCCA C -WAP
W2 F2 TGCTAAGCTTTGCC AGCCAGTGTCCTGGCCAG C -WAP
W2 F3 TGCTAAGCTTTGCC CAGCTCGGCCTCTGTCGGGAC C -WAP
W2R1 CCTTCTGCAGG GAAATTGGGAGTGACACAGGA C -WAP
W2R2 CCTTCTGCAGG GTCCACCTGGCACTGGTCC C -WAP
W2R3 CCTTCTGCAGG ATTGCGGCAGCATTTCATCTG C -WAP

The HE4 fragments were separately amplified from 0,5 µ 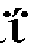 of cDNA in a reaction mixture containing 1 µM of each forward and reverse primer, 75 mM Tris-HCl (pH 8.8 at 25°C), 20 mM (NH₄)₂SO₄, 0.1% (v/v) Tween 20, 2 mM MgCl₂, 0.02 u/µ 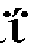 Taq-polymerase (Abgene, Surrey, UK) and 0.1 mM of each deoxynucleotide in a final volume of 25 µ 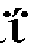 with the following temperature cycle repeated 30 times: 30 seconds incubations at 95°C, 50°C and 72°C.
PCR products and f88-4, digested with Hindlll and Pstl, were ligated together and transfected into E. coli JM109 where after clones were selected on LB plates with tetracycline. Two clones of each construct were amplified in E. coli JM109 and double-stranded DNA was prepared for DNA sequencing. DNA sequencing was performed using the Big dye terminator v1.1 cycle sequencing kit and a f88-4 vector specific primer. Sequencing reactions were sent to CyberGene AB (Huddinge, Sweden) for analysis. Sequence raw data was analyzed using the free software Chromas version 1.45 (Technelysium Pty Ltd., Australia). Nucleotide sequencing verified insertion in frame with the leader peptide and the mature phage coat protein pVIII. The HE4 inserts demonstrated identity to the expected HE4 fragment sequences (accession number AY212888). The positions of the HE4 fragments expressed as fusion proteins with coat protein

contacted with the HE4-coated well, and the anti-HE4 antibody in the sample is bound and captured. After binding and washing to remove non-specifically bound immune complexes, the antibody-antigen complex is detected. Detection may be carried out with any suitable method, such as the addition of a second antibody linked to a label.

In accordance with various embodiments of the methods of this aspect of the invention, an anti-HE4 antibody reference value may be obtained from a control group of apparently healthy subjects, for example, as described in EXAMPLES 2 and 3. In some embodiments, the antibody reference value is determined in an ELISA assay using serum obtained from healthy subjects diluted at least 1:20. In one embodiment, the antibody reference value is determined using serum obtained from patients diagnosed with and/or previously treated for a cancer comprising HE4-expressing tumor cells. An exemplary ELISA assay for detecting anti-HE4 antibody levels in blood samples is described in EXAMPLE 2.

In accordance with the prognostic applications of the invention, in one embodiment the level of anti-HE4 antibody in a biological sample obtained from an ovarian cancer patient is then compared to the antibody reference value. If the antibody concentration in the patient tested is higher than the reference value, such as at least 1.5-fold, more preferably at least two-fold or higher, with a P value of less than 0.05, and the patient has previously undergone treatment for ovarian cancer, then the patient has a reduced likelihood of recurrence of ovarian cancer. If the antibody concentration in an ovarian cancer patient is lower than the reference value, such as at least 1.5-fold or two-fold or lower, with a P value of less than 0.05, and the patient has previously undergone treatment for ovarian cancer, then the patient has an increased likelihood of recurrence of ovarian cancer. In another embodiment, the presence of anti-HE4 antibody is determined by comparison to a negative antibody control sample and optionally also to a positive antibody control sample.

In another aspect, the specification provides a method of assessing the prognosis of a human cancer patient suffering from an HE4-expressing tumor. The method comprises: (a) determining the presence or amount of anti-HE4 antibodies in a biological sample from a human patient suffering from an HE4-expressing tumor by contacting the biological sample with a polypeptide encoded by a polynucleotide that selectively hybridizes to a sequence that is at least 80% identical, such as at least 90% identical to a sequence comprising at least 20 contiguous nucleotides of SEQ ID NO:1; (b) determining the presence or amount of soluble HE4-related peptides (SHRP) encoded by a polynucleotide that selectively hybridizes to a sequence at least 80%, such as at least 90% identical to a sequence comprising at least 20 contiguous nucleotides of SEQ ID NO:1 in a biological sample from the human patient tested in step (a); and (c) comparing the amount of anti-HE4 antibodies determined in step (a) to an antibody reference level, and comparing the amount of SHRP determined in step (b) to an antigen reference level, wherein the detection of SHRP in the sample at a lower amount than the antigen reference level, in combination with the detection of anti-HE4 antibodies in the sample at a higher amount than the antibody reference level, is indicative of a good prognosis for the patient.

In accordance with this aspect, the method comprises the step of determining the presence and/or amount of SHRP in a biological sample obtained from a patient suffering from an HE4-expressing tumor, such as an ovarian cancer patient. As described above, SHRP is a soluble protein that has been found in the circulation of both healthy and cancer patients. The presence or amount of SHRP may be determined using any assay capable of detecting and/or measuring the amount of SHRP polypeptide.

In one embodiment, the concentration of an SHRP polypeptide encoded by a polynucleotide that selectively hybridizes to a sequence at least 80% identical (e.g., at least 85% identical, or at least 90% identical, or at least 95% identical, or at least 99% identical) to SEQ ID NO:1, or a fragment thereof comprising at least 20 consecutive nucleotides (or at least 25 or 30, or at least 40, 60, or 80 consecutive nucleotides) of SEQ ID NO:1, is measured in the biological sample.

In another embodiment, the amount of an SHRP polypeptide at least 80% identical (e.g., at least 85% identical, or at least 90% identical, or at least 95% identical, or at least 99% identical) to the human soluble HE4-related protein provided as SEQ ID NO:2, or a fragment thereof comprising at least 10 consecutive amino acid residues (or at least 20 or at least 30, such as at least 50 consecutive amino acid residues) of SEQ ID NO:2 is measured in the biological sample.

The concentration and/or relative amount, or detection of soluble HE4-related protein (SHRP) present in a biological fluid sample may be determined using any convenient method for measuring SHRP including, but not limited to, ELISA, radioimmunoassay, chemiluminescence assay, immunofluorescence staining and the like that include an antibody that specifically binds to SHRP. Other protein detection methods may also be used to measure SHRP, including mass spectroscopy, western blot, FACS, and the like. Suitable biological samples include a biological fluid selected from the group consisting of blood, plasma, serum, ascitic fluid, and urine.

Specific antibodies, including monoclonal antibodies directed against SHRP and variants thereof, can be readily prepared using conventional techniques, and may be used in such methods. For example, a double determinant ("sandwich") ELISA assay using two MAbs 2H5 and 3D8 (which recognize two different epitopes on the same antigen) may be used to detect SHRP in sera, as described in Hellstrom, I., et al., Cancer Research 63:3695-3700 (2003). Other ELISA assays may be used to detect one or more variants of HE4 using antibodies described above, or other antibodies against HE4.

In accordance with various embodiments of the methods of this aspect, an SHRP antigen reference value may be obtained from a control group of apparently healthy subjects; for example, as described in Example 3. In some embodiments, the antigen reference value is determined in an ELISA assay using serum obtained from healthy subjects. For example, in a serum sample, the serum may be diluted up to 1:100 and measured in an ELISA assay, where a negative control obtained from a healthy subject gives an absorbance value of <0.2 and a positive control obtained from a ovarian cancer patient gives an absorbance value of >0.2. See Hellstrom, I., et al., Cancer Research 63:3695-3700 (2003). Absorbance values may be determined by any method known in the art. For example, absorbance of light at 450 nanometers, often referred to as the optical density (OD), is commonly used. In one embodiment, the antigen reference value is determined using serum obtained from patients diagnosed with and/or previously treated for a cancer comprising HE4-expressing tumor cells.

In some embodiments, the methods further comprise the step of determining levels of another ovarian cancer marker, such as integrin-linked kinase (INK), CA125, TADG-12, kallikrein 10, prostasin, osteopontin, creatine kinase beta, serotransferrin, neutrophil-gelatinase associated lipocalin (NGAL), CD163, or Gc-globulin in a biological sample obtained from the subject. The second marker may be detected at the DNA, RNA, or protein level using conventional methods known in the art.

In another aspect, the specification provides a method of monitoring the efficacy of treatment of a human patient diagnosed with an HE4-expressing tumor. The method comprises: (a) determining a first concentration of HE4-mesothelin antibodies in a first biological sample taken from a human patient diagnosed with an HE4-expressing tumor prior to initiation of treatment for cancer; (b) determining a second concentration of anti-HE4 antibodies in a second biological sample from the human patient taken after initiation of treatment for cancer; and (c) comparing the first and second concentrations of anti-HE4 antibodies, wherein an increase in the second concentration of anti-HE4 antibodies as compared to the first concentration of anti-HE4 antibodies measured in the first biological sample indicates a positive response to the treatment for cancer.

In accordance with the method of this aspect, a first biological sample is taken from a cancer patient before initiation of treatment, and a second biological sample is taken from the patient at least one time after initiation of treatment. In some embodiments, plural treated biological samples from the subject (e.g., a subject in a preclinical trial) are taken over periodic intervals of time after initiation of treatment.

As used herein, the term "treatment" refers to surgical intervention or to the administration of one or more cancer inhibitory agents for the alleviation of symptoms associated with cancer, or halt of further progression or worsening of the symptoms. For example, successful treatment may include a removal of a tumor, such as an HE4-expressing tumor; an alleviation of symptoms or halting the progression of the disease, as measured by a reduction in the growth rate of a tumor; a halt in the growth of a tumor; a reduction in size of the tumor; partial or complete remission of the cancer; or increased survival or clinical benefit. For example, treatment of a subject suffering from an HE4-expressing tumor may include one or more of the following: surgery to remove one or more tumors and/or administration of a therapeutic agent, such as chemotherapy, radiation therapy, protein therapeutics (e.g., antibodies, gene therapy, cancer vaccine therapy, stem cell transplant, or other therapy).

For example, with regard to treatment for ovarian cancer, surgery is a preferred treatment. The type of surgery depends upon how widespread the cancer is when diagnosed (the cancer stage), as well as the type and grade of cancer. The surgeon may remove one (unilateral oophorectomy) or both ovaries (bilateral oophorectomy), the fallopian tubes (salpingectomy), and the uterus (hysterectomy). For some very early tumors (stage 1, low grade or low-risk disease), only the involved ovary and fallopian tube will be removed (called a "unilateral salpingo-oophorectomy," or "USO"), especially in young females who wish to preserve their fertility. In advanced stages of disease, as much tumor as possible is removed (debulking surgery). In cases where this type of surgery is successful, the prognosis is improved compared to patients where large tumor masses (more than 1 cm in diameter) are left behind. Chemotherapy is typically used after surgery to treat any residual disease. Chemotherapeutic agents, such as a platinum derivative (e.g., taxane) may be administered systemically, or may be administered intra-peritoneally via direct infusion into the abdominal cavity. Other examples of therapeutic agents for use in treatment of ovarian cancer include, but are not limited to, protein therapeutics (e.g., antibodies), gene therapy, cancer vaccine therapy, and stem cell transplants. The methods of this aspect of the invention may also be used to measure the efficacy of candidate therapeutic agents for treatment of ovarian cancer.

The methods of this aspect may also be used to determine the clinical status of a patient after undergoing a treatment, such as surgery to remove a tumor. In accordance with this embodiment, the level of anti-HE4 antibody in a biological sample obtained from a cancer patient that has been treated for an HE4-expressing tumor is then compared to the antibody reference value. If the antibody concentration in the patient tested is higher than the reference value, such as at least 1.5-fold, more preferably at least two-fold or higher, with a P value of less than 0.05, then the patient's clinical status is expected to be improved with the treatment (i.e., the patient has a reduced likelihood of recurrence of ovarian cancer). If the antibody concentration in the treated cancer patient is lower than the reference value, such as at least 1.5-fold or two-fold or lower, with a P value of less than 0.05, then the patient's clinical status is not expected to be improved with the treatment (i.e., the patient has an increased likelihood of recurrence of ovarian cancer).

In another aspect, a kit is provided for detecting the presence of HE4-expressing cells in a human subject. The kit comprises reagents specific for detection of anti-HE4 antibodies in a biological sample obtained from a human subject and printed instructions for comparison of the detected presence or amount of anti-HE4 antibodies with a reference standard. The methods for detection of anti-HE4 antibodies described herein may be performed using the kits. In one example, the kit comprises a detection reagent for detecting anti-HE4 antibodies comprising an HE4 polypeptide encoded by a polynucleotide that selectively hybridizes to a sequence that is at least 80% identical, or at least 90% identical, or at least 95% identical, or at least 98% identical, or at least 99% identical to a sequence comprising at least 20 contiguous nucleotides of SEQ ID NO:1. In some example, the kit comprises a detection reagent for detecting anti-HE4 antibodies comprising an HE4 polypeptide that is at least 80% identical, or at least 90% identical, or at least 95% identical, or at least 98% identical, or at least 99% identical to an amino acid sequence comprising at least 10 contiguous amino acids of SEQ ID NO:2. In some examples, the kit comprises HE4 polypeptide, or fragment thereof, that is immobilized onto a solid matrix, such as, for example, a polystyrene or polycarbonate microwell or dipstick, a membrane, or a glass support (e.g., a glass slide). For example, an HE4-coated well of an ELISA plate may be utilized, wherein the biological sample is contacted with the HE4-coated well and the anti-HE4 antibody in the sample is bound and captured.

In some examples, the kit further comprises a reference standard selected from the group consisting of a specific numerical threshold, a negative control sample for concurrent evaluation, or statistical information correlating the amount of anti-HE4 antibodies detected with the likelihood of the presence of HE4-expressing cancer cells in the subject. In some examples, the reference standard is a negative control sample, and wherein the negative control sample is included in the kit.

In preferred examples, the methods and kits are capable of use at a point-of-care location, such as a medical clinic (e.g., doctor's office) or hospital, in order to rapidly obtain test results. Point-of-care testing (POCT) refers to any hospital or medical clinic (doctor's office) employee performing any type of laboratory test outside of the central laboratory. POCT has revolutionized the continuum of patient care process by providing laboratory results efficiently at the patient's bedside for various tests such as HIV testing, urine dipstick, etc. For example, rapid tests to detect HIV antibodies have been developed that demonstrate sensitivities and specificities comparable to those of enzyme immunoassays without the need for sophisticated laboratory equipment and highly-trained technicians. POCT can be used with unprocessed whole blood or oral fluid specimens. See Branson, B.M., J. Lab. Medicine 27(7/8):288-295 (2003). POCT assays may be in any assay format that allows for rapid testing, such as particle agglutination, immunoconcentration and immunochromatography.

For example, particle agglutination POCT assays for detecting anti-HE4 antibodies may be carried out by mixing a patient specimen containing anti-HE4 antibodies with latex particles coated with HE4 polypeptide (antigen), and if anti-HE4 antibody is present, cross-linking occurs within 10 to 60 minutes and results in agglutination, with results interpreted visually.

In another example of a POCT assay format for detecting anti-HE4 antibodies, an immunoconcentration device (flow through) may be used which employs solid-phase capture technology, which involves the immobilization of HE4 polypeptides (antigen) on a porous membrane. The patient specimen flows through the membrane and is absorbed into an absorbent pad. If anti-HE4 antibodies are present in the specimen, a dot or a line visibly forms on the membrane when developed with a signal reagent (e.g., a colloidal gold or selenium conjugate). A procedural control may also be included on the membrane.

In yet another example of a POCT assay format to detect anti-HE4 antibodies, immunochromatographic (lateral flow) strips may be used that incorporate both antigen (HE4) and signal reagent into a nitrocellulose strip. The patient specimen is applied to an absorbent pad, or the specimen may be diluted in a vial of buffer into which the test device is inserted. The specimen migrates through the strip and combines with the signal reagent. A positive reaction results in a visual line on the membrane where the HE4 antigen has been applied. A procedural control line may be applied to the strip beyond the HE4 antigen line.

The following examples merely illustrate the best mode now contemplated for practicing the invention, but should not be construed to limit the invention.

### EXAMPLE 1

This Example describes the production and purification of recombinant Human Epididymis Protein 4 (HE4) protein in Chinese Hamster Ovary (CHO) cells.

### Methods:

### HE4-CIHDpa plasmid construction

Recombinant HE4 protein (SEQ ID NO:2) was generated as follows.

A cDNA fragment encoding the Human Epididymis Protein 4 (HE4) (SEQ ID NO:2) was amplified by high fidelity polymerase chain-reaction using the following primers:
The forward sense primer:
   5'-AAAAACCGGTATGCCTGCTTGTCGCCTAGG-3', (SEQ ID NO:3) was designed to introduce an *Age I* restriction enzyme recognition site at the 5' end of the gene appropriate for cloning.
The reverse antisense primer:
   5'-AAAACCTGCAGGTCAGAAATTGGGAGTGACAC-3', (SEQ ID NO: 4), was designed to introduce an Sbf I restriction enzyme recognition site at the 3' end of the gene appropriate for cloning.

The amplified DNA fragment containing the HE4 cDNA was digested with *Age I* and *Sbf I*, and cloned into the CIHDpa mammalian expression vector (by replacing the IFN-γ with the HE4 fragment). The CIHDpa vector, provided by Dr. Say Kong Ng of the National University of Singapore, utilizes destabilizing sequences on a selection marker for improved recombinant protein productivity in CHO-DB44 cells, as described in Ng et al., Metab Eng 9:304 (2007)), incorporated herein by reference. Briefly described, the CIHDpa expression vector contains a standard cytomegalovirus (CMV) promoter upstream of the cDNA insertion site. A herpes simplex virus thymidine kinase (HSV-th) promoter is positioned upstream of a dihydrofolate reductase (dhfr) gene, which serves as a selective marker for successful transfectants (i.e., cells deficient in dhfr require hypoxanthine and thymidine supplements to survive). Immediately downstream of the dhfr marker is a murine ornithine decarboxylase PEST (MODC PEST) region and a series of AU-rich elements (ARE), which are expressed as fusion components at the carboxy terminal end of the dhfr protein. The MODC PEST peptide serves as a degradation signal leading to instability of the marker protein. The ARE region serves to destabilize the mRNA encoding the marker protein. With the dhfr marker destabilized at the transcription and translation levels, only transfected cells that have highly efficient production of the plasmid-encoded proteins will survive, thus leading to improved productivity of the recombinant HE4 protein after selection.

The resulting expression vector containing HE4, designated "HE4-CIHDpa," was confirmed by restriction enzyme analysis, and the inserted HE4 cDNA was also confirmed by sequencing the complete cDNA insert.

### Transfection of HE4-CIHDpa plasmid into Chinese Hamster Ovary (CHO) cells

Dihydrofolate reductase deficient (DHFR-) Chinese Hamster Ovary (CHO-DG44) cells were grown in serum-free CHO medium (Hyclone, Logan, Utah; SH30333) at 37°C, 5% C0₂. 0.5 million or 1 million CHO-DG44 cells were seeded in each well of a 24-well plate with 500 µl serum-free medium per well and were incubated overnight at the conditions described above. 1 µg or 5 µg of endo-toxin free HE4-CIHDpa plasmid was combined with 2 µl Lipofectamine™ 2000 (Invitrogen) in 100 µl Opti-MEM serum-free medium. After incubating for 20 minutes, the DNA-Lipofectamine™ complexes were added into each well containing CHO-DG44 cells. The cells were then transferred into hypoxanthine and thymidine ("HT"). HT-supplemented HyQ PF-CHO medium and allowed to recover for 2 days before transferring to HyQ PF-CHO medium without the HT supplement to select for transfectants. After 28 days in the selective medium, 1 million cells transfected with 1 µg plasmid grew and recovered with a cell viability > 95%.

### Production and Purification of HE4 protein by transfected CHO cells

HE4 protein production in culture supernatants from CHO cells transfected with HE4-CIHDpa plasmid was evaluated by using a commercially available kit, marketed by Fujirebio Diagnostics, Inc., which is based on a double determinant (Sandwich) ELISA assay, as described in Hellstrom I., et al., Cancer Research 63:3695-3700 (2003), incorporated herein by reference.

As described in Hellstrom et al. (2003), a monoclonal Ab 3D8 (specific for one epitope on HE4) was coated overnight onto the wells of an assay plate, after which 100 µl supernatant from the CHO culture transfected with HE4-CIHDpa plasmid was added to the cells and incubated for 1 hour. The assay plate was then incubated with a biotinylated second anti-HE4 monoclonal Ab, 2H5 (specific for a different HE4 epitope) for 1 hour.

TMB (3,3',5,5'-tetramethylbenzidine), a chromogenic substrate for Peroxidase (KPL, Gaithersburg, MD) was added and permitted to incubate for 15 minutes. Optical density (OD) readings were made at 450 nm. The OD450 from CHO supernatant was 3.1, as compared with 0.078 for medium alone, indicating that HE4 protein was highly expressed by the CHO cells and secreted into the culture medium.

### Purification of HE4 recombinant protein by affinity chromatography

The recombinant HE4 protein was purified from high producing lines of CHO cells transfected with HE4-CIHDpa plasmid by affinity chromatography as follows.

Anti-HE4 monoclonal Abs 3D8 and 2H5 were coupled to 6-aminohexanoic acid N-hydroxysuccinimide ester-activated-Sepharose 4B (Sigma, St. Louis, Missouri; #A9019). Briefly described, 0.5 g of powder was swollen in 1 mM HCl for 15 minutes. The beads were drained and resuspended in 3 mg anti-HE4 monoclonal Ab in 1 ml of 0.5 M NaCl/0.1 M NaHC0₃ (pH 8.3) solution. The column was stored at 4°C overnight. The next day, the resin was blocked with 1 M Tris-HCl for 2 hours. The unbound anti-HE4 monoclonal Ab was removed by washing the resin with phosphate-buffered saline (PBS). Supernatant from large scale cultures of CHO cells transfected with HE4-CIHDpa plasmid was collected and the pH was adjusted with NaHC0₃ to pH 8.0. The adjusted cell supernatant was applied to the column, and bound HE4 protein was eluted with 0.1 M glycine-HCl (pH 2.7) and neutralized with 200 µl 2M Tris-HCl. The eluted HE4 protein was concentrated with a 10 kD centrifugal filter tube (Millipore, Billerica, MA). The column purified HE4 recombinant protein was assayed by Sandwich ELISA, which was performed as described above. The results of the ELISA assays are shown below in TABLE 1.

**TABLE 1: Presence of HE4 protein as determined by Sandwich ELISA.**

| **Samples** | **Dilution factor** | **OD450** |
|---|---|---|
| Non-purified CHO supernatant (i.e. before column purification) | undiluted | 2.832 |
| CHO medium control | undiluted | 0.110 |
| Column-Purified HE4 protein (0.1 µg) | undiluted | 3.432 |

The column purified human recombinant HE4 protein obtained from CHO cells was obatined as described above and pooled for use in developing an ELISA assay to detect anti-HE4 antibody in patient samples, as described in EXAMPLE 2. The recombinant human HE4 protein was also tested in ELISA assays for binding to mouse anti-HE4 antibodies, and it was determined that the mouse anti-HE4 antibodies recognized both native and recombinant human HE4 protein (data not shown).

### Conclusion:

These results demonstrate the successful cloning, expression, and purification of recombinant human HE4 protein from CHO cells.

### EXAMPLE 2

This Example describes the successful development of an ELISA assay capable of detecting naturally occurring human anti-HE4 antibodies in human serum.

### Methods:

### Development of an ELISA Assay for Detecting anti-HE4 antibodies:

An ELISA assay was developed for detecting/measuring the amount of anti-HE4 antibodies in serum as follows. 0.6 µg/mL of purified recombinant human HE4 protein (produced as described in EXAMPLE 1), was coated overnight onto the wells of an ELISA assay plate. Matching wells were left uncoated as controls for background. After blocking for 2 hours with 3% BSA/PBS, human sera or plasma at dilutions of 1:20 and 1:40 (obtained as described below) were added to all of the wells on the ELISA assay plate. All dilutions of both samples and reagents were performed in 3% BSA/PBS.

After incubating with HRP-conjugated mouse anti-human IgG antibody and TMB substrate, the assay plate was scanned with a Dynatech MR 5000 plate reader at 450 nm. The background control (stickiness due to the innate nature of IgG to stick non-specifically to the control wells of the ELISA assay plate) was measured as OD450 in the parallel control wells (to which no antigen had been attached), and the background control values were subtracted from the reading in the coated wells to give the final IgG level.

### Plasma Samples Obtained from Ovarian Cancer Patients

A pilot study was performed in which plasma samples were obtained from ten ovarian cancer patients, most of whom had advanced (stage III-IV) disease, and one healthy female subject, and tested in the anti-HE4 ELISA assay in order to test for the presence of naturally occurring anti-HE4 antibodies. The human plasma samples were added to each well of the ELISA plate at 1:20 and 1:40 dilutions and were incubated at room temperature for 1 hour. The wells were washed with PBS-Tween 20, and then 1:1000 diluted HRP-conjugated mouse anti-human IgG antibody (Invitrogen, Carlsbad, California) was added to each well and incubated for 1 hour at room temperature. After washing the plate again with PBS-Tween 20, SureBlue™ TMB Microwell Peroxidase Substrate (KPL) was added to each well and incubated for 15 minutes at room temperature, after which the interaction was terminated by adding the TMB stop solution (KPL). Optical density (OD) at 450 nanometers was measured with a DynaTech MR 5000 plate reader (DynaTech Laboratories, Inc.).

### Results:

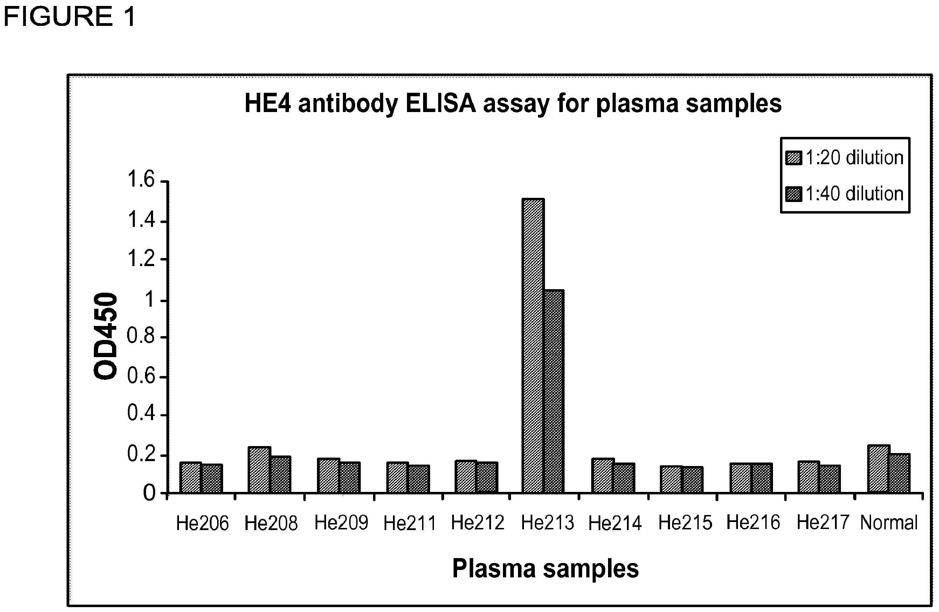

The results of the ELISA assay testing for the presence/amount of anti-HE4 antibodies from the human sera samples are presented in FIGURE 1. As shown in FIGURE 1, one of the ten patients with ovarian carcinoma (subject He213) had an OD450 ≥ 1.0 for both dilutions, thus indicating the presence of anti-HE4 antibodies. It is noted that the plasma from patient He213 (with plasma that was positive for anti-HE4 abs), was found to be negative for the presence of HE4 antigen using the assay to detect the presence of HE4 antigen as described in U.S. Patent No. 7,270,960, incorporated herein by reference (data not shown). However, cultured tumor cells derived from the tumor of patient He213 were shown to express the HE4 antigen (data not shown).

### Conclusion:

The results described in this example demonstrate the successful development of an ELISA assay capable of detecting naturally occurring human anti-HE4 antibodies in human serum. The results also demonstrate the presence of anti-HE4 antibodies in a subject with ovarian cancer. Based on our previous study on antibodies to mesothelian, (see Hellstrom I. et al., Cancer Epidemiol Biomarkers Prev. 17(6):1520-1526 (2008)), it is expected that the presence of, or an increase in titer of antibodies to HE4 identified in ovarian cancer patients that are undergoing, or having undergone treatment (via surgery, chemotherapy, radiation and/or immunotherapy), either alone, or in combination with a finding of a decreased amount of HE4 antigen (as compared to the baseline amount at the start of the treatment), or no circulating HE4 antigen, will indicate that the cancer therapy has been effective in damaging the tumor. It is further expected that a decrease in the titer of anti-HE4 antibodies, or no detectable anti-HE4 antibodies to HE4 in ovarian cancer patients undergoing treatment, either alone, or in combination with increased amount of circulating HE4 antigen (as compared to the baseline amount at the start of treatment), will indicate that the cancer therapy has been ineffective in damaging the tumor, and/or indicative of a relapse.

### EXAMPLE 3

This Example describes a study in which the ELISA assay developed for detecting the presence/amount of antibodies to HE4 (described in EXAMPLE 2) was used to titrate the amount of anti-HE4 antibody present in serum from 3 patients with ovarian carcinoma, and 20 healthy female control subjects.

### Methods:

### Serum Samples Obtained from Ovarian Cancer Patients

As shown in TABLE 2 below, serum samples from three patients with early stage (I/II) ovarian carcinoma and 20 healthy human female subjects were obtained at the Fred Hutchinson Cancer Research Center (Seattle, Washington), 4 of which are shown in Table 2 below. Sera were drawn from the subjects and asssayed using the methods as described in Example 2.

### Results:

The sera obtained as described above were assayed at 1:20 and 1:40 dilutions using the ELISA method described in EXAMPLE 2. The results are summarized in TABLE 2 below.

**TABLE 2: Results of HE4 antibody analysis of serum from three ovarian Cancer patients, and 4 healthy controls using the ELISA assay to detect/measure HE4 antibodies.**

| **Patient Code** | **Clinical Disease/Condition** | **OD450 > 0.500** (at least in the 1:20 dilution |
|---|---|---|
| 100C17 | Ovarian Carcinoma | + |
| 206241 | Ovarian Carcinoma | + |
| 208448 | Ovarian Carcinoma | + |
| 1 | healthy female control | - |
| 2 | healthy female control | - |
| 3 | healthy female control | - |
| 4 | healthy female control | - |
| 5-24 (total of 24 healthy female control subjects) | Healthy female control | 3 positive/21 negative (of 24 total tested) |

As summarized in TABLE 2, all three serum samples from patients with ovarian carcinoma, and three of the 24 serum samples from healthy female subjects had an OD450 ≥ 0.5 for the 1:20 dilution. As shown in TABLE 2, all of the three patients with ovarian cancer had anti-HE4 antibodies in their serum, as indicated by an OD450 ≥ 0.5 at the 1:20 dilution. These results are consistent with the results described in Example 2, and further demonstrate the successful development of an ELISA assay capable of detecting naturally occurring human anti-HE4 antibodies in human serum. The results also demonstrate the presence of anti-HE4 antibodies in a subjects with both early and later stage ovarian cancer.

### EXAMPLE 4

### Reactivity with HE4 fragments displayed as phage fusion proteins

The epitope specificity of the human Anti HE4 antibodies was determined by testing the reactivity of human samples that displayed anti HE4 reactivity towards HE4 fragments expressed as fusion proteins with phage coat protein pVIII in a phage ELISA.

### Cloning of HE4 fragments in f88-4

cDNA, prepared from mRNA isolated from OvCar-3 cells, served as template for PCR amplification of the gene parts coding for the HE4 domains for cloning in the phage display vector f88-4. PCR primer pairs, listed in Table 3, were constructed for amplification of the coding regions indicated in Figure x. In the 5'-ends were restriction sites for HindIII and PstI inserted for cloning in fusion with the pVIII signal peptide and the pVIII mature coat protein.

**Table 3. PCR primers used for amplification of HE4 fragments**

| Primer | Sequence 5' to 3' | WAP |
|---|---|---|
| W1F1 | TGCTAAGCTTTGCC GAGAAGACTGGCGTGTGCCC | N-WAP |
| W1F2 | TGCTAAGCTTTGCC AGCGAATGCGCCGACAACC | N-WAP |
| W1F3 | TGCTAAGCTTTGCC GACCAGAACTGCACGCAAG | N-WAP |
| W1R1 | CCTTCTGCAGG ATCATTGGGCAGAGAGCAG | N-WAP |
| W1R2 | CCTTCTGCAGG GTCCGAGACGCACTCTTGC | N-WAP |
| W1R3 | CCTTCTGCAGG GCTGCAGCACTTGAGGTTG | N-WAP |
| W2F1 | TGCTAAGCTTTGCC AAGGAGGGTTCCTGCCCCCA | C-WAP |
| W2F2 | TGCTAAGCTTTGCC AGCCAGTGTCCTGGCCAG | C-WAP |
| W2F3 | TGCTAAGCTTTGCC CAGCTCGGCCTCTGTCGGGAC | C-WAP |
| W2R1 | CCTTCTGCAGG GAAATTGGGAGTGACACAGGA | C-WAP |
| W2R2 | CCTTCTGCAGG GTCCACCTGGCACTGGTCC | C-WAP |
| W2R3 | CCTTCTGCAGG ATTGCGGCAGCATTTCATCTG | C-WAP |

The HE4 fragments were separately amplified from 0,5 µl of cDNA in a reaction mixture containing 1 µM of each forward and reverse primer, 75 mM Tris-HCl (pH 8.8 at 25°C), 20 mM (NH₄)₂SO₄, 0.1% (v/v) Tween 20, 2 mM MgCl₂, 0.02 u/µl Taq-polymerase (Abgene, Surrey, UK) and 0.1 mM of each deoxynucleotide in a final volume of 25 µl with the following temperature cycle repeated 30 times: 30 seconds incubations at 95°C, 50°C and 72°C.

PCR products and f88-4, digested with HindIII and PstI, were ligated together and transfected into *E. coli* JM109 where after clones were selected on LB plates with tetracycline. Two clones of each construct were amplified in *E. coli* JM109 and double-stranded DNA was prepared for DNA sequencing. DNA sequencing was performed using the Big dye terminator v1.1 cycle sequencing kit and a f88-4 vector specific primer. Sequencing reactions were sent to CyberGene AB (Huddinge, Sweden) for analysis. Sequence raw data was analyzed using the free software Chromas version 1.45 (Technelysium Pty Ltd., Australia). Nucleotide sequencing verified insertion in frame with the leader peptide and the mature phage coat protein pVIII. The HE4 inserts demonstrated identity to the expected HE4 fragment sequences (accession number AY212888). The positions of the HE4 fragments expressed as fusion proteins with coat protein pVIII using the primers shown in Table1 is shown in Figure 2. The amino acid numbers refer to positions is the HE4 amino acid sequence set forth in SEQ ID NO: 2.

### Phage ELISA

Sequence verified phage clones were amplified, purified, concentrated with PEG/NaCl and were diluted in PBS for use as coating antigen in the direct phage ELISA assay, or alternatively in in 1%BSA in PBS as antigen in the sandwhich phage ELISA.

In the direct phage ELISA the HE4 fragment phages were diluted in carbonate buffer pH 9.2 and coated in microtiter wells. Binding of the human anti HE4 ab's was determined after dilution of the patient serum in PBS-1%BSA and incubation in HE4 phage coated plates. The bound hIg was determined by incubation with HRP Anti hIg Ab.

In the sandwich HE4 phage ELISA the patient samples were diluted with PBS-1%BSA and incubated in microtiter plates coated with anti-Human IgG for adsorption of the hIgG. The coated plates were incubated with the different HE4 pVIII phage particles in a volume of 100 µl/well were added. After two hours incubation, wells were washed and a rabbit anti-M13 antibody (established in-house) was added. After incubation and washing, a HRP labelled swine anti rabbit antibody (Dako) was added. After the final wash TMB substrate was added and the plate was measured at 620 nm after 5 minute incubation. wt M-13 phage was used as negative control n both ELISA assays.

### SEQUENCE LISTING

SEQ ID NO:1: HE4 cDNA (human). 486bp, CDS: 38-418
   GenBank: AY212888.1 Homo sapiens HE4 protein (WFDC2) mRNA, complete cds
SEQ ID NO:2: HE4 protein (human) (124aa)
   GenBank: AAO52683.1 HE4 protein [Homo sapiens]
SEQ ID NO: 3: (forward primer)
   5'-AAAAACCGGTATGCCTGCTTGTCGCCTAGG-3
SEQ ID NO:4 (reverse primer)
   5'-AAAACCTGCAGGTCAGAAATTGGGAGTGACAC-3'

## Claims

1. A method for detecting the presence of HE4-expressing cells in a human subject comprising determining the presence or amount of anti-HE4 antibodies in a biological sample obtained from the human subject, wherein the biological sample is a biological fluid selected from the group consisting of blood, plasma, serum, ascitic fluid, urine, saliva, tears, pleural fluid, sputum, vaginal fluid, and washings obtained during a medical procedure, and wherein the presence or amount of anti-HE4 antibodies in the biological sample is indicative of the presence of HE4- expressing cells in the human subject, and wherein the presence of HE4-expressing cells is indicative that the subject is suffering from or at risk for developing ovarian cancer.

2. The method of claim 1, wherein the presence or amount of anti-HE4 antibodies in the biological sample is determined by contacting the biological sample with a polypeptide encoded by a polynucleotide that selectively hybridizes to a sequence at least 90% identical to a sequence comprising at least 20 contiguous nucleotides of SEQ ID NO: 1.

3. The method of claim 1, further comprising comparing the determined amount of anti-HE4 antibodies to a reference standard, wherein an amount of anti-HE4 antibody detected that is greater than the reference standard is indicative of the presence of HE4-expressing cells in the human subject.

4. The method of claim 1, further comprising performing at least one additional diagnostic assay to determine if the subject with anti-HE4 antibodies has ovarian cancer.

5. The method of claim 1, wherein binding of the polypeptide to an anti-HE4 antibody comprises detecting a signal selected from the group consisting of a radionuclide, a fluorophore, a binding event between an avidin molecule and a biotin molecule, a binding event between a streptavidin molecule and a biotin molecule, and a product of an enzyme reaction.

6. The method of claim 1 wherein the human subject is undergoing therapeutic treatment for a cancer associated with HE4-expressing tumor cells.

7. The method of claim 2, wherein the amount of anti-HE4 antibodies is determined using an ELISA assay.

8. The method of claim 1, further comprising determining the presence or amount of soluble HE4-related peptides (SHRP) in the biological sample, wherein the presence or amount of anti-HE4 antibodies in the biological sample in combination with the presence or amount of soluble HE4-related peptides is indicative of the presence of HE4-expressing cells in the human subject.

## Patentansprüche

1. Verfahren zum Nachweis des Vorhandenseins von HE4-exprimierenden Zellen in einem menschlichen Subjekt, das das Bestimmen des Vorhandenseins oder der Menge von Anti-HE4-Antikörpern in einer vom menschlichen Subjekt erhaltenen biologischen Probe umfasst, wobei die biologische Probe eine biologische Flüssigkeit ist, die aus der Gruppe ausgewählt ist, die aus Blut, Plasma, Serum, Ascitesflüssigkeit, Urin, Speichel, Tränen, Pleuralflüssigkeit, Sputum, Vaginalflüssigkeit und Spülungen, die während eines medizinischen Verfahrens erhalten wurden, besteht, wobei das Vorhandensein oder die Menge von Anti-HE4-Antikörpern in der biologischen Probe das Vorhandensein von HE4-exprimierenden Zellen im menschlichen Subjekt anzeigt, und wobei das Vorhandensein von HE4-exprimierenden Zellen anzeigt, dass das Subjekt an Eierstockkrebs leidet oder das Risiko besteht, diesen zu entwickeln.

2. Verfahren nach Anspruch 1, wobei das Vorhandensein oder die Menge von Anti-HE4-Antikörpern in der biologischen Probe bestimmt wird, indem die biologische Probe mit einem Polypeptid in Kontakt gebracht wird, das durch ein Polynukleotid codiert wird, das selektiv zu einer Sequenz hybridisiert, die zu mindestens 90 % identisch mit einer Sequenz ist, die mindestens 20 zusammenhängende Nukleotide der SEQ ID Nr. 1 umfasst.

3. Verfahren nach Anspruch 1, ferner umfassend: Vergleichen der bestimmten Menge von Anti-HE4-Antikörpern mit einem Referenzstandard, wobei eine Menge von nachgewiesenen Anti-HE4-Antikörpern, die größer als der Referenzstandard ist, das Vorhandensein von HE4-exprimierenden Zellen im menschlichen Subjekt anzeigt.

4. Verfahren nach Anspruch 1, ferner umfassend: Durchführen mindestens eines zusätzlichen diagnostischen Tests, um zu bestimmen, ob das Subjekt mit Anti-HE4-Antikörpern Eierstockkrebs hat.

5. Verfahren nach Anspruch 1, wobei ein Binden des Polypeptids an einen Anti-HE4-Antikörper ein Nachweisen eines Signals umfasst, das aus der Gruppe ausgewählt ist, die aus einem Radionuklid, einem Fluorophor, einem Bindungsvorgang zwischen einem Avidinmolekül und einem Biotinmolekül, einem Bindungsvorgang zwischen einem Streptavidinmolekül und einem Biotinmolekül und einem Produkt einer Enzymreaktion besteht.

6. Verfahren nach Anspruch 1, wobei das menschliche Subjekt sich einer therapeutischen Behandlung einer mit HE4-exprimierenden Tumorzellen assoziierten Krebserkrankung unterzieht.

7. Verfahren nach Anspruch 2, wobei die Menge von Anti-HE4-Antikörpern unter Verwendung eines ELISA-Tests bestimmt wird.

8. Verfahren nach Anspruch 1, ferner umfassend: Bestimmen des Vorhandenseins oder der Menge von löslichen HE4-bezogenen Peptiden (SHRP) in der biologischen Probe, wobei das Vorhandensein oder die Menge von Anti-HE4-Antikörpern in der biologischen Probe in Kombinationen mit dem Vorhandensein oder der Menge von löslichen HE4-bezogenen Peptiden das Vorhandensein von HE4-exprimierenden Zellen im menschlichen Subjekt anzeigt.

## Revendications

1. Procédé de détection de la présence de cellules exprimant HE4 dans un sujet humain comprenant la détermination de la présence ou de la quantité d'anticorps anti-HE4 dans un échantillon biologique prélevé sur le sujet humain, dans lequel l'échantillon biologique est un liquide biologique choisi parmi le groupe constitué de sang, plasma, sérum, liquide d'ascite, urine, salive, larmes, liquide pleural, expectoration, liquide vaginal et lavages obtenus pendant une intervention médicale, et dans lequel la présence ou la quantité d'anticorps anti-HE4 dans l'échantillon biologique est indicatrice de la présence de cellules exprimant HE4 dans le sujet humain, et dans lequel la présence de cellules exprimant HE4 est indicatrice que le sujet est atteint de cancer de l'ovaire ou à risque d'en développer.

2. Procédé de la revendication 1, dans lequel la présence ou la quantité d'anticorps anti-HE4 dans l'échantillon biologique est déterminée en mettant en contact l'échantillon biologique avec un polypeptide codé par un polynucléotide qui s'hybride sélectivement à une séquence au moins 90 % identique à une séquence comprenant au moins 20 nucléotides contigus de SEQ ID NO: 1.

3. Procédé de la revendication 1, comprenant en outre la comparaison de la quantité déterminée d'anticorps anti-HE4 à un standard de référence, dans lequel une quantité d'anticorps anti-HE4 détectée qui est supérieure au standard de référence est indicatrice de la présence de cellules exprimant HE4 dans le sujet humain.

4. Procédé de la revendication 1, comprenant en outre la réalisation d'au moins un test de diagnostic supplémentaire pour déterminer si le sujet avec des anticorps anti-HE4 a un cancer de l'ovaire.

5. Procédé de la revendication 1, dans lequel la liaison du polypeptide à un anticorps anti-HE4 comprend la détection d'un signal choisi parmi le groupe constitué par un radionucléide, un fluorophore, un évènement de liaison entre une molécule d'avidine et une molécule de biotine, un évènement de liaison entre une molécule de streptavidine et une molécule de biotine et un produit d'une réaction enzymatique.

6. Procédé de la revendication 1 dans lequel le sujet humain est sous traitement thérapeutique pour un cancer associé à des cellules tumorales exprimant HE4.

7. Procédé de la revendication 2, dans lequel la quantité d'anticorps anti-HE4 est déterminée par la technique ELISA.

8. Procédé de la revendication 1, comprenant en outre la détermination de la présence ou de la quantité de peptides solubles apparentés à HE4 (SHRP) dans l'échantillon biologique, dans lequel la présence ou la quantité d'anticorps anti-HE4 dans l'échantillon biologique en association avec la présence ou la quantité de peptides solubles apparentés à HE4 est indicatrice de la présence de cellules exprimant HE4 dans le sujet humain.


### SEQUENCE LISTING

<110> FUJIREBIO DIAGNOSTICS, INC. HELLSTROM, Karl Erik HELLSTROM, Ingegerd LIU, Pu JAFFAR, Jade SWISHER, Elizabeth
<120> METHODS FOR DETECTING ANTI-HE4 ANTIBODIES AND METHODS OF DIAGNOSIS AND/OR PROGNOSIS OF CONDITIONS ASSOCIATED WITH HE4-EXPRESSING CELLS
<130> D4496-00164
<140> PCT/US11/49274
   <141> 2011-08-26
<150> US 61/377,387
   <151> 2010-08-26
<160> 38
<170> PatentIn version 3.3
<210> 1
   <211> 486
   <212> DNA
   <213> Homo sapiens
<400> 1
   tgagagaaag cggccgcacc ccgcccggca tagcaccatg cctgcttgtc gcctaggccc   60
   gctagccgcc gccctcctcc tcagcctgct gctgttcggc ttcaccctag tctcaggcac  120
   aggagcagag aagactggcg tgtgccccga gctccaggct gaccagaact gcacgcaaga  180
   gtgcgtctcg gacagcgaat gcgccgacaa cctcaagtgc tgcagcgcgg gctgtgccac  240
   cttctgctct ctgcccaatg ataaggaggg ttcctgcccc caggtgaaca ttaactttcc  300
   ccagctcggc ctctgtcggg accagtgcca ggtggacagc cagtgtcctg gccagatgaa  360
   atgctgccgc aatggctgtg ggaaggtgtc ctgtgtcact cccaatttct gagctccggc  420
   caccaccagg ctgagcagtg aagatagaaa gtttctgcct ggccctgcag cgtgttacag  480
   cccacc                                                             486
<210> 2
   <211> 124
   <212> PRT
   <213> Homo sapiens
<400> 2
   Met Pro Ala Cys Arg Leu Gly Pro Leu Ala Ala Ala Leu Leu Leu Ser
   1               5                   10                  15
   Leu Leu Leu Phe Gly Phe Thr Leu Val Ser Gly Thr Gly Ala Glu Lys
               20                  25                  30
   Thr Gly Val Cys Pro Glu Leu Gln Ala Asp Gln Asn Cys Thr Gln Glu
           35                  40                  45
   Cys Val Ser Asp Ser Glu Cys Ala Asp Asn Leu Lys Cys Cys Ser Ala
       50                  55                  60
   Gly Cys Ala Thr Phe Cys Ser Leu Pro Asn Asp Lys Glu Gly Ser Cys
   65                  70                  75                  80
   Pro Gln Val Asn Ile Asn Phe Pro Gln Leu Gly Leu Cys Arg Asp Gln
                   85                  90                  95
   Cys Gln Val Asp Ser Gln Cys Pro Gly Gln Met Lys Cys Cys Arg Asn
               100                 105                 110
   Gly Cys Gly Lys Val Ser Cys Val Thr Pro Asn Phe
           115                 120
<210> 3
   <211> 30
   <212> DNA
   <213> Artificial Sequence
<220>
   <223> Forward sense primer
<400> 3
   aaaaaccggt atgcctgctt gtcgcctagg 30
<210> 4
   <211> 32
   <212> DNA
   <213> Artificial Sequence
<220>
   <223> Reverse antisense primer
<400> 4
   aaaacctgca ggtcagaaat tgggagtgac ac 32
<210> 5
   <211> 45
   <212> PRT
   <213> Homo sapiens
<400> 5
   Glu Lys Thr Gly Val Cys Pro Glu Leu Gln Ala Asp Gln Asn Cys Thr
   1               5                   10                  15
   Gln Glu Cys Val Ser Asp Ser Glu Cys Ala Asp Asn Leu Lys Cys Cys
               20                  25                  30
   Ser Ala Gly Cys Ala Thr Phe Cys Ser Leu Pro Asn Asp
           35                  40                  45
<210> 6
   <211> 49
   <212> PRT
   <213> Homo sapiens
<400> 6
   Lys Glu Gly Ser Cys Pro Gln Val Asn Ile Asn Phe Pro Gln Leu Gly
   1               5                   10                  15
   Leu Cys Arg Asp Gln Cys Gln Val Asp Ser Gln Cys Pro Gly Gln Met
               20                  25                  30
   Lys Cys Cys Arg Asn Gly Cys Gly Lys Val Ser Cys Val Thr Pro Asn
           35                  40                  45
   Phe
<210> 7
   <211> 22
   <212> PRT
   <213> Homo sapiens
<400> 7
   Glu Lys Thr Gly Val Cys Pro Glu Leu Gln Ala Asp Gln Asn Cys Thr
   1               5                   10                  15
   Gln Glu Cys Val Ser Asp
               20
<210> 8
   <211> 22
   <212> PRT
   <213> Homo sapiens
<400> 8
   Asp Gln Asn Cys Thr Gln Glu Cys Val Ser Asp Ser Glu Cys Ala Asp
   1               5                   10                  15
   Asn Leu Lys Cys Cys Ser
               20
<210> 9
   <211> 23
   <212> PRT
   <213> Homo sapiens
<400> 9
   Ser Glu Cys Ala Asp Asn Leu Lys Cys Cys Ser Ala Gly Cys Ala Thr
   1               5                   10                  15
   Phe Cys Ser Leu Pro Asn Asp
               20
<210> 10
   <211> 25
   <212> PRT
   <213> Homo sapiens
<400> 10
   Lys Glu Gly Ser Cys Pro Gln Val Asn Ile Asn Phe Pro Gln Leu Gly
   1               5                   10                  15
   Leu Cys Arg Asp Gln Cys Gln Val Asp
               20                  25
<210> 11
   <211> 14
   <212> PRT
   <213> Homo sapiens
<400> 11
   Gln Leu Gly Leu Cys Arg Asp Gln Cys Gln Val Asp Ser Gln
   1               5                   10
<210> 12
   <211> 14
   <212> PRT
   <213> Homo sapiens
<400> 12
   Gln Leu Gly Leu Cys Arg Asp Gln Cys Gln Val Asp Ser Gln
   1               5                   10
<210> 13
   <211> 24
   <212> PRT
   <213> Homo sapiens
<400> 13
   Ser Gln Cys Pro Gly Gln Met Lys Cys Cys Arg Asn Gly Cys Gly Lys
   1               5                   10                  15
   Val Ser Cys Val Thr Pro Asn Phe
               20
<210> 14
   <211> 48
   <212> PRT
   <213> Homo sapiens
<400> 14
   Ser Glu Cys Ala Asp Asn Leu Lys Cys Cys Ser Ala Gly Cys Ala Thr
   1               5                   10                  15
   Phe Cys Ser Leu Pro Asn Asp Lys Glu Gly Ser Cys Pro Gln Val Asn
               20                  25                  30
   Ile Asn Phe Pro Gln Leu Gly Leu Cys Arg Asp Gln Cys Gln Val Asp
           35                  40                  45
<210> 15
   <211> 14
   <212> DNA
   <213> Artificial Sequence
<220>
   <223> Forward sense primer
<400> 15
   tgctaagctt tgcc 14
<210> 16
   <211> 20
   <212> DNA
   <213> Artificial Sequence
<220>
   <223> Reverse antisense primer
<400> 16
   gagaagactg gcgtgtgccc 20
<210> 17
   <211> 14
   <212> DNA
   <213> Artificial Sequence
<220>
   <223> Forward sense primer
<400> 17
   tgctaagctt tgcc 14
<210> 18
   <211> 19
   <212> DNA
   <213> Artificial Sequence
<220>
   <223> Reverse antisense primer
<400> 18
   agcgaatgcg ccgacaacc 19
<210> 19
   <211> 14
   <212> DNA
   <213> Artificial Sequence
<220>
   <223> Forward sense primer
<400> 19
   tgctaagctt tgcc 14
<210> 20
   <211> 19
   <212> DNA
   <213> Artificial Sequence
<220>
   <223> Reverse antisense primer
<400> 20
   gaccagaact gcacgcaag 19
<210> 21
   <211> 11
   <212> DNA
   <213> Artificial Sequence
<220>
   <223> Forward sense primer
<400> 21
   ccttctgcag g 11
<210> 22
   <211> 19
   <212> DNA
   <213> Artificial Sequence
<220>
   <223> Reverse antisense primer
<400> 22
   atcattgggc agagagcag 19
<210> 23
   <211> 11
   <212> DNA
   <213> Artificial Sequence
<220>
   <223> Forward sense primer
<400> 23
   ccttctgcag g 11
<210> 24
   <211> 19
   <212> DNA
   <213> Artificial Sequence
<220>
   <223> Reverse antisense primer
<400> 24
   gtccgagacg cactcttgc 19
<210> 25
   <211> 11
   <212> DNA
   <213> Artificial Sequence
<220>
   <223> Forward sense primer
<400> 25
   ccttctgcag g 11
<210> 26
   <211> 19
   <212> DNA
   <213> Artificial Sequence
<220>
   <223> Forward sense primer
<400> 26
   gctgcagcac ttgaggttg 19
<210> 27
   <211> 14
   <212> DNA
   <213> Artificial Sequence
<220>
   <223> Forward sense primer
<400> 27
   tgctaagctt tgcc 14
<210> 28
   <211> 20
   <212> DNA
   <213> Artificial Sequence
<220>
   <223> Reverse antisense primer
<400> 28
   aaggagggtt cctgccccca 20
<210> 29
   <211> 14
   <212> DNA
   <213> Artificial Sequence
<220>
   <223> Forward sense primer
<400> 29
   tgctaagctt tgcc 14
<210> 30
   <211> 18
   <212> DNA
   <213> Artificial Sequence
<220>
   <223> Reverse antisense primer
<400> 30
   agccagtgtc ctggccag 18
<210> 31
   <211> 14
   <212> DNA
   <213> Artificial Sequence
<220>
   <223> Forward sense primer
<400> 31
   tgctaagctt tgcc 14
<210> 32
   <211> 21
   <212> DNA
   <213> Artificial Sequence
<220>
   <223> Reverse antisense primer
<400> 32
   cagctcggcc tctgtcggga c 21
<210> 33
   <211> 11
   <212> DNA
   <213> Artificial Sequence
<220>
   <223> Forward sense primer
<400> 33
   ccttctgcag g 11
<210> 34
   <211> 21
   <212> DNA
   <213> Artificial Sequence
<220>
   <223> Reverse antisense primer
<400> 34
   gaaattggga gtgacacagg a 21
<210> 35
   <211> 11
   <212> DNA
   <213> Artificial Sequence
<220>
   <223> Forward sense primer
<400> 35
   ccttctgcag g 11
<210> 36
   <211> 19
   <212> DNA
   <213> Artificial Sequence
<220>
   <223> Reverse antisense primer
<400> 36
   gtccacctgg cactggtcc 19
<210> 37
   <211> 11
   <212> DNA
   <213> Artificial Sequence
<220>
   <223> Forward sense primer
<400> 37
   ccttctgcag g 11
<210> 38
   <211> 21
   <212> DNA
   <213> Artificial Sequence
<220>
   <223> Reverse antisense primer
<400> 38
   attgcggcag catttcatct g 21